# EUROPÄISCHE PATENTSCHRIFT

(11) **EP 3 415 173 B1**
(45) Veröffentlichungstag und Bekanntmachung des Hinweises auf die Patenterteilung: **13.05.2020**
(21) Anmeldenummer: 18162929.6
(22) Anmeldetag: 20.03.2018
(51) Int. Cl.: A61L 27/30, A61L 31/08

(54) **OBERFLÄCHENBESCHICHTUNG FÜR EIN MEDIZINISCHES INSTRUMENT, MEDIZINISCHES INSTRUMENT MIT EINER OBERFLÄCHENBESCHICHTUNG UND VERFAHREN ZUM HERSTELLEN EINER OBERFLÄCHENBESCHICHTUNG FÜR EIN MEDIZINISCHES INSTRUMENT**
SURFACE COATING FOR A MEDICAL INSTRUMENT, MEDICAL INSTRUMENT WITH A SURFACE COATING AND METHOD FOR PRODUCING A SURFACE COATING FOR A MEDICAL INSTRUMENT
REVÊTEMENT DE SURFACE POUR UN INSTRUMENT MÉDICAL, INSTRUMENT MÉDICAL DOTÉ D'UN REVÊTEMENT DE SURFACE ET PROCÉDÉ DE FABRICATION D'UN REVÊTEMENTS DE SURFACE POUR UN INSTRUMENT MÉDICAL

(30) Priorität: 13.06.2017 DE 102017112941
(43) Veröffentlichungstag der Anmeldung: 19.12.2018
(73) Patentinhaber: IMT Masken und Teilungen AG, 8606 Greifensee (CH)
(72) Erfinder: THORWARTH, Götz, 8600 Dübendorf (CH)
(74) Vertreter: Pleyer, Hans Anno

(56) Entgegenhaltungen:
- EP-A1- 0 560 534
- EP-A1- 0 774 618
- WO-A1-2006/098813
- DATABASE WPI Week 201577 Thomson Scientific, London, GB; AN 2015-69977U XP002786001, & JP 2015 198910 A (SAKURA KUREPASU KK) 12. November 2015 (2015-11-12)

## Beschreibung

Die Erfindung betrifft ein medizinisches Instrument mit einer Oberflächenbeschichtung.

Hierbei geht es um eine Oberflächenbeschichtung für ein medizinisches Instrument, das für eine Verwendung unter sterilen Bedingungen, z.B. für therapeutische Anwendungen am menschlichen oder tierischen Körper, vorgesehen ist. Im Folgenden sollen unter dem Begriff "medizinisches Instrument" nicht etwa nur chirurgische Handinstrumente, wie z.B. Skalpelle, verstanden werden, sondern jegliche Art von Vorrichtungen, die für medizinische Zwecke bestimmt sind und demensprechend steril zu halten sind. Insbesondere sollen auch Vorrichtungen, die zur Implantation in den menschlichen oder tierischen Körper vorgesehen sind, unter den Begriff "medizinisches Instrument" fallen. Ferner sollen vorliegend auch mikrofluidische Vorrichtungen (z.B. sogenannte "Flow Cells") wie Sie beispielsweise für diagnostische Zwecke und insbesondere für die DNA-Sequenzierung verwendet werden, mit dem Begriff "medizinisches Instrument" bezeichnet werden.

In der Medizintechnik sind sogenannte "single-use"-Produkte weit verbreitet. Darunter versteht man medizinische Instrumente, die aus verschiedenen Gründen (z.B. unzureichende Sterilisierbarkeit, Materialänderungen durch klinische Aufbereitung oder die Auslegung der mechanischen Dauerfestigkeit) nicht für einen mehrfachen Einsatz vorgesehen sind. Eine - z.B. aus Kostengründen - dennoch erfolgende Mehrfachverwendung an einem Patienten kann für diesen ein gesundheitliches Risiko darstellen.

Hieraus ergibt sich der Bedarf nach einer Markierung auf derartigen für den "single use" vorgesehenen medizinischen Instrumenten, welche eine unzulässige Mehrfachverwendung anzeigt.

Die JP 2015 198 819 A beschreibt ein medizinisches Instrument mit einer Warnanzeige, welche auf einem Aufdruck aus einer farbveränderlichen Tinte beruht. Wenn das medizinische Instrument zur erneuten Verwendung einer Sterilisationsbehandlung unterzogen wird, ändert die Tinte ihre Farbe von violett zu blau, so dass ein Warnhinweis lesbar wird.

An der bekannten Lösung ist nachteilig, dass die Warnanzeige gesondert an dem medizinischen Instrument angebracht ist und sie dementsprechend die Dimensionierung des Instruments und/oder seine mechanischen Eigenschaften an der betreffenden Stelle ändert und ggf. beeinträchtigt. Somit muss für die Markierung an dem Instrument Platz bereitgestellt werden, welcher dann nicht mehr für die funktionale Ausgestaltung des Instruments zur Verfügung steht. Auch ist denkbar, dass eine solche tintenbasierte Warnanzeige unbeabsichtigt oder beabsichtigt von dem medizinischen Instrument entfernt wird.

Aufgabe der Erfindung ist es daher, eine Markierung zur Detektion einer nicht vorgesehenen Mehrfachverwendung eines medizinischen Instruments bereitzustellen, welche ihre Anzeigefunktion in zuverlässiger und irreversibler Weise erfüllt und dabei keine oder nur eine insignifikante dimensionale Änderung des Instruments oder seiner mechanischen Eigenschaften mit sich bringt. Zudem soll die Markierung entsprechend den üblichen Anforderungen im medizinischen Bereich biokompatibel sein.

Diese Aufgabe wird erfindungsgemäß durch die Schaffung eines medizinischen Instrument mit einer Oberflächenbeschichtung mit den Merkmalen des Anspruchs 1 gelöst.

Demnach umfasst das medizinische Instrument eine Oberflächenbeschichtung umfassend ein Interferenzfilter mit wenigstens einer dielektrischen Schicht und wenigstens einer metallischen Schicht, die übereinander angeordnet sind. Dabei weist die Oberflächenbeschichtung in einem ersten Zustand ein erstes spektrales Reflexionsvermögen auf. Die wenigstens eine metallische Schicht und/oder die wenigstens eine dielektrische Schicht ist ausgebildet, durch den Einfluss einer korrosiven Umgebung auf die Oberflächenbeschichtung strukturell verändert zu werden, wodurch die Oberflächenbeschichtung in einen zweiten Zustand überführbar ist. In dem zweiten Zustand weist die Oberflächenbeschichtung ein zweites spektrales Reflexionsvermögen auf, welches von dem ersten spektralen Reflexionsvermögen verschieden ist. Das Interferenzfilter umfasst eine Mehrzahl von dielektrischen Schichten und/oder eine Mehrzahl von metallischen Schichten.

Die Erfindung geht von dem Gedanken aus, dass durch korrosive Einflüsse ein oder mehrere metallische Schichten und/oder eine oder mehrere dielektrische Schichten in einem metall-dielektrischen Interferenzfilter aus mehreren Festkörperschichten derart veränderbar sind, dass eine irreversible Farbänderung eintritt. Hierfür werden eine oder mehrere transparente dielektrische Schichten (z.B. aus Titandioxid) und eine oder mehrere metallische Schichten (z.B. Titan sowie das korrosionsstabile Tantal) dergestalt in einer Multilagenschicht kombiniert, dass sich ein Interferenzfilter mit einem ersten spektralen Reflexionsvermögen ergibt. Durch einen klinischen Aufbereitungsprozess des medizinischen Instruments - oder durch seinen erstmaligen bestimmungsgemäßen Gebrauch, wie z.B. bei einer mikrofluidischen Vorrichtung - wird die Oberflächenbeschichtung einer korrosiven Umgebung ausgesetzt. Hierdurch werden ein oder mehrere metallische Schichten oxidiert und somit transparent und/oder es werden ein oder mehrere metallische oder dielektrische Schichten delaminiert oder aufgelöst. In der Folge hat das Interferenzfilter durch seinen geänderten Aufbau bzw. die geänderten optischen Eigenschaften der wenigstens einen Schicht ein zweites spektrales Reflexionsvermögen, d.h. die Oberflächenbeschichtung vermittelt nun einen anderen Farbeindruckt als zuvor.

Dem Begriff "korrosive Umgebung" soll vorliegend die in der Chemie gängige Definition zu Grunde gelegt werden, wonach Korrosion eine chemische Reaktion oder eine elektrochemische Reaktion eines Werkstoffes mit Stoffen aus seiner Umgebung ist, bei welcher eine messbare Veränderung am Werkstoff eintritt. Eine korrosive Umgebung ist demnach eine Umgebung, die Korrosion ermöglicht oder fördert. Dabei kann der Einfluss der korrosiven Umgebung beispielsweise dadurch ausgeübt werden, dass die Oberflächenbeschichtung in Kontakt mit einem korrosiven Medium gebracht wird.

Als metallische Schichten der erfindungsgemäßen Vorrichtung eignen sich somit beispielsweise solche Metalle und Legierungen, welche z.B. unter typischen Bedingungen der klinischen Aufbereitung korrosiv zersetzen - wie z.B. Magnesium (Mg), Eisen (Fe) oder Calcium (Ca) - bzw. ein transparentes Oxid bilden, wie z.B. Aluminium (Al), Titan (Ti), Zirkonium (Zr), Niob (Nb), Molybdän (Mo), Tantal (Ta) oder Wolfram (W). Gemäß einer Weiterbildung kann dabei auch eine Kombination eines Metalls mit geringer Beständigkeit und eines Metall mit hoher Beständigkeit vorgesehen sein. Für den medizinischen Bereich können beispielsweise die Kombinationen Titan/Tantal, Titan/Wolfram oder Aluminium/Niob zum Einsatz kommen, da in jenen Kombinationen metallische Elemente im Schichtverband eine klinische Reprozessierung unoxidiert überdauern können.

Die verschiedenen Schichten des Interferenzfilters können mittels an sich bekannter Beschichtungsverfahren in kommerziell erhältlichen Anlagen mit der erforderlichen Genauigkeit hergestellt werden. So kommen je nach konkreter Anwendung beispielsweise verschiedene Varianten der physikalischen oder chemischen Gasphasenabscheidung in Betracht, wie z.B. reaktives Magnetronsputtern, reaktives Lichtbogenverdampfen, Elektronenstrahl-Verdampfen oder eine plasmaunterstützte chemische Gasphasenabscheidung.

Eine mittels derartiger Beschichtungsverfahren direkt auf ein medizinisches Instrument aufgebrachte Markierung kann so ausgelegt sein, dass sie den Anforderungen an die Biokompatibilität im medizinischen Bereich genügt und überdies keine unerwünschte signifikante dimensionale Änderung des medizinischen Instruments mit sich bringt.

Es kann in einer Ausführungsform beispielsweise vorgesehen sein, dass eine vertikale Gesamtdicke der Oberflächenbeschichtung weniger als 1000 nm, wie z.B. weniger als 600 nm oder sogar weniger als 500 nm beträgt.

In einer Ausführungsform besteht die mindestens eine metallische Schicht aus Titan. Eine solche Schicht kann beispielsweise durch eine Sterilisationsbehandlung, wie z.B. einer Behandlung mit einer Base, zu Titandioxid oxidieren und optisch transparent werden.

Es kann auch vorgesehen sein, dass die mindestens eine metallische Schicht aus Magnesium besteht. Eine Magnesium Schicht kann unter dem Einfluss der korrosiven Umgebung, z.B. bei einer Sterilisationsbehandlung mittels Autoklavierung, oxidieren und in der Folge delaminieren.

Die mindestens eine dielektrische Schicht kann beispielsweise aus Titandioxid bestehen. Titandioxid ist optisch transparent und eignet sich somit als Schichtkomponente in einem metall-dielektrischen Interferenzfilter.

In einer Ausführungsform hat die Oberflächenbeschichtung bei Beleuchtung mit weißem Licht in dem ersten Zustand eine erste Farbe und in dem zweiten Zustand eine zweite Farbe, wobei die zweite Farbe von der ersten Farbe verschieden ist. Beispielsweise kann die erste Farbe im grün-blauen Spektralbereich liegen, und die zweite Farbe kann im orange-roten Spektralbereich liegen (oder umgekehrt).

Es kann ferner vorgesehen sein, dass das erste spektrale Reflexionsvermögen ein erstes Reflexionsmaximum bei einer ersten Wellenlänge aufweist und das zweite spektrale Reflexionsvermögen ein zweites Reflexionsmaximum bei einer zweiten Wellenlänge aufweist, wobei eine Differenz zwischen der zweiten Wellenlänge und der ersten Wellenlänge dem Betrag nach wenigstens 100 nm, wie z.B. wenigstens 150 nm, oder sogar wenigstens 200 nm ist. Auf diese Weise kann eine für das menschliche Auge deutlich wahrnehmbare Farbänderung der Oberflächenbeschichtung in Folge des Aussetzens der korrosiven Umgebung sichergestellt werden. Beispielsweise kann die erste Wellenlänge im roten Spektralbereich liegen, und die zweite Wellenlänge kann im grün-blauen Spektralbereich liegen.

Gemäß einer Variante ist die wenigstens eine metallische Schicht ausgebildet, durch den Einfluss der korrosiven Umgebung oxidiert zu werden, wodurch die Oberflächenbeschichtung in den zweiten Zustand überführbar ist. Beispielsweise kann die metallische Schicht ausgebildet sein, durch Oxidation dielektrisch und transparent zu werden, wodurch sich die optischen Eigenschaften des Interferenzfilters verändern.

Alternativ oder zusätzlich kann die wenigstens eine metallische Schicht ausgebildet sein, durch den Einfluss der korrosiven Umgebung von einer anderen Schicht der Oberflächenbeschichtung abgelöst zu werden, wodurch die Oberflächenbeschichtung in den zweiten Zustand überführbar ist. So kann die metallische Schicht beispielsweise durch den Einfluss der korrosiven Umgebung (z.B. bei einer Sterilisationsbehandlung) delaminieren und - ggf. zusammen mit darüber angeordneten weiteren Schichten - gänzlich entfernt werden.

Es liegt auch im Rahmen der Erfindung, dass die wenigstens eine dielektrische Schicht ausgebildet sein kann, durch den Einfluss der korrosiven Umgebung von einer anderen Schicht der Oberflächenbeschichtung abgelöst zu werden, wodurch die Oberflächenbeschichtung in den zweiten Zustand überführbar ist. Es ist beispielsweise denkbar, dass sich eine dielektrische Schicht aus einem wasserlöslichen transparenten Salz, wie z.B. Natriumchlorid (NaCl), unter dem Einfluss der korrosiven Umgebung auflöst, so dass ggf. auch darüber angeordnete weitere Schichten delaminiert werden.

Gemäß einer Ausführungsform ist die Oberflächenbeschichtung durch eine Sterilisationsbehandlung, bei welcher die Oberflächenbeschichtung der korrosiven Umgebung ausgesetzt wird, in den zweiten Zustand überführbar.

In einer Variante umfasst die Sterilisationsbehandlung dabei einen Autoklavierprozess. Beispielsweise ist die Sterilisationsbehandlung ein Autoklavierprozess. Die Oberflächenbeschichtung kann z.B. bei dem Autoklavierprozess für eine Dauer von 1 bis 30 Minuten Temperaturen im Bereich von 121 °C bis 135 °C bei einem Absolutdruck von 1 bis 5 bar ausgesetzt sein. Derartige Bedingungen können die korrosive Umgebung zum Überführen der Oberflächenbeschichtung in den zweiten Zustand bereitstellen.

Alternativ oder zusätzlich kann die Sterilisationsbehandlung eine Behandlung der Oberflächenbeschichtung mit einer Base, z.B. mit einer Base mit einem pH-Wert, der größer als 10 ist, umfassen. Beispielsweise kann die Sterilisationsbehandlung eine Behandlung der Oberflächenbeschichtung mit einer Base, z.B. mit einer Base mit einem pH-Wert, der größer als 10 ist, sein.

Die mindestens eine metallische Schicht kann ausgebildet sein, durch den Einfluss der korrosiven Umgebung ihre optische Transparenz zu erhöhen oder von einem optisch intransparenten Zustand in einen optisch transparenten Zustand überführt zu werden. Dies kann beispielsweise durch Oxidation erfolgen, wobei die metallische Schicht auch zu einer transparenten dielektrischen Schicht werden kann.

Erfindungsgemäß umfasst das Interferenzfilter eine Mehrzahl von dielektrischen Schichten und/oder eine Mehrzahl von metallischen Schichten. Mittels eines derartigen Stapels einer Mehrzahl von metallischen und/oder dielektrischen Schichten lassen sich die jeweiligen Farbeigenschaften der Oberflächenbeschichtung in dem ersten Zustand und in dem zweiten Zustand vorteilhaft gestalten. So können beispielsweise zunächst die für das jeweilige medizinische Instrument geeigneten Materialien (entsprechend den mechanischen Anforderungen und den Anforderungen an die Biokompatibilität etc.) ausgewählt werden. Sodann kann ein erster Stapel aus mehreren Schichten dieser Materialien entworfen werden, der durch seine Interferenzeigenschaften dahingehend optimiert ist, einen ersten Farbeindruck hervorzurufen. Ferner kann ein zweiter Stapel entworfen werden, der für einen zweiten Farbeindruck, der von dem ersten Farbeindruck verschieden ist, optimiert ist. Nun können die beiden Entwürfe durch einen Abgleich der Schichtdicken oder eine Stapelung mit einer delaminierenden Zwischenschicht miteinander kombiniert werden, so dass unter dem Einfluss der korrosiven Umgebung der erste Farbeindruck in den zweiten Farbeindruck übergeht.

Die Aufgabe wird erfindungsgemäß durch ein medizinisches Instrument gelöst, welches mit einer Oberflächenbeschichtung versehen ist. Das mit Bezug auf die Oberflächenbeschichtung gesagte gilt insoweit für das erfindungsgemäße medizinische Instrument und umgekehrt.

Bei einer Ausführungsform ist das medizinische Instrument ein medizinisches Handinstrument, wie z.B. ein chirurgisches Handinstrument, oder ein Teil davon, wie z.B. ein Skalpell oder ein chirurgischer Bohrer. Alternativ kann es sich bei dem medizinischen Instrument aber auch um eine Vorrichtung handeln, die zur Implantation in den menschlichen oder tierischen Körper vorgesehen ist, wie z.B. ein Zwischenwirbelkäfig ("lumbaler Cage"). Es ist auch denkbar, dass das medizinische Instrument eine mikrofluidische Vorrichtung, wie z.B. eine sogenannte "Flow Cell" ist. Derartige mikrofluidische Vorrichtungen werden häufig im Rahmen von molekularmedizinischen Verfahren für diagnostische Zwecke, wie z.B. bei der DNA-Sequenzierung, eingesetzt. Beim erstmaligen bestimmungsgemäßen Gebrauch und/oder bei einer Sterilisationsbehandlung einer derartigen mikrofluidischen Vorrichtung kann die Oberflächenbeschichtung einer korrosiven Umgebung ausgesetzt sein.

Beschrieben wird auch ein Verfahren zum Herstellen einer Oberflächenbeschichtung für ein medizinisches Instrument. Das Verfahren umfasst dabei das Bereitstellen eines medizinischen Instruments mit einer Oberfläche und das Erzeugen eines Interferenzfilters auf der Oberfläche, wobei der Interferenzfilter wenigstens eine dielektrische Schicht und wenigstens eine metallischen Schicht umfasst, die übereinander angeordnet sind. Die Oberflächenbeschichtung weist dabei in einem ersten Zustand ein erstes spektrales Reflexionsvermögen auf und die wenigstens eine metallische Schicht und/oder die wenigstens eine dielektrische Schicht ist ausgebildet, durch den Einfluss einer korrosiven Umgebung auf die Oberflächenbeschichtung strukturell verändert zu werden, wodurch die Oberflächenbeschichtung in einen zweiten Zustand überführbar ist. Dabei weist die Oberflächenbeschichtung in dem zweiten Zustand ein zweites spektrales Reflexionsvermögen auf, welches von dem ersten spektralen Reflexionsvermögen verschieden ist. Das Interferenzfilter umfasst eine Mehrzahl von dielektrischen Schichten und/oder eine Mehrzahl von metallischen Schichten.

Der der Erfindung zugrundeliegende Gedanke soll nachfolgend anhand der in den Figuren dargestellten Ausführungsbeispiele näher erläutert werden.

Es zeigen:
- Figur 1A: einen Ausschnitt einer vertikalen Querschnittsansicht einer Oberflächenbeschichtung in einem ersten Zustand;
- Figur 1B: die Oberflächenbeschichtung aus Figur 1A in einem zweiten Zustand nach einer Sterilisationsbehandlung;
- Figur 2A: einen Ausschnitt einer vertikalen Querschnittsansicht einer Oberflächenbeschichtung in einem ersten Zustand;
- Figur 2B: die Oberflächenbeschichtung aus Figur 2A in einem zweiten Zustand nach einer Sterilisationsbehandlung;
- Figur 3A: das grafisch aufgetragene spektrale Reflexionsvermögen der Oberflächenbeschichtung aus Figur 2A;
- Figur 3B: das grafisch aufgetragene spektrale Reflexionsvermögen der Oberflächenbeschichtung aus Figur 2B;
- Figur 4A: einen Ausschnitt einer vertikalen Querschnittsansicht einer Oberflächenbeschichtung in einem ersten Zustand;
- Figur 4B: die Oberflächenbeschichtung aus Figur 4A in einem zweiten Zustand nach einer Sterilisationsbehandlung.
- Figur 5A: einen Ausschnitt einer vertikalen Querschnittsansicht einer Oberflächenbeschichtung in einem ersten Zustand; und
- Figur 5B: die Oberflächenbeschichtung aus Figur 5A in einem zweiten Zustand nach einer Sterilisationsbehandlung.

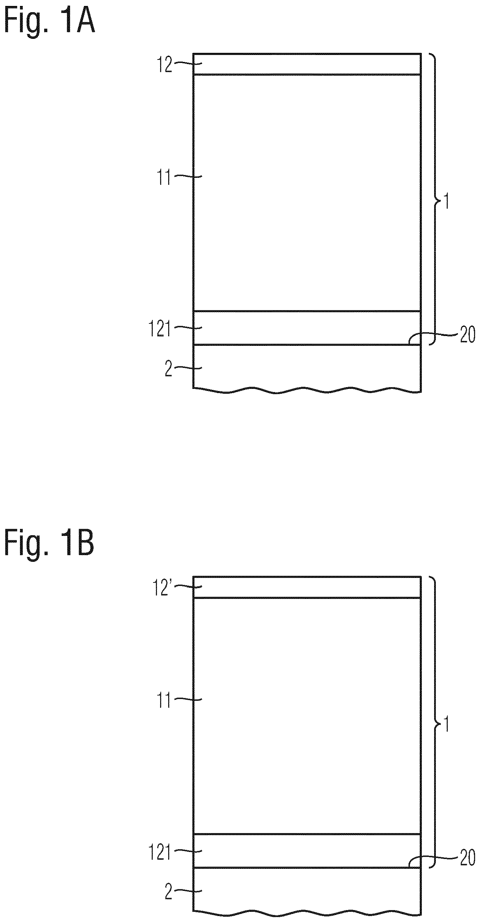

Die Figur 1A zeigt einen Ausschnitt einer vertikalen Querschnittsansicht einer Oberflächenbeschichtung 1 für ein medizinisches Instrument 2 gemäß einem ersten Ausführungsbeispiel. Das medizinische Instrument 2 kann dabei ein "single-use"-Implantat zum Einbringen in den menschlichen Körper, wie z.B. ein Zwischenwirbelkäfig ("lumbaler Cage"), sein. Die Oberflächenbeschichtung 1 ist auf einer Oberfläche 20 des medizinischen Instruments 1 angeordnet. Die Oberfläche 20 kann beispielsweise von einer Substratschicht aus einem thermoplastischen Kunststoff wie Polyetheretherketon (PEEK) ausgebildet sein.

Die Oberflächenbeschichtung 1 kann beispielsweise mittels Elektronenstrahlverdampfen auf der Oberfläche 20 erzeugt werden. Dies kann in einem maskierten Prozess erfolgen, so dass die Oberflächenbeschichtung 1 z.B. in Form eines warnenden Schriftzugs der Art "DO NOT USE" aufgebracht werden kann.

In dem in der Figur 1A dargestellten ersten Zustand umfasst die Oberflächenbeschichtung 1 einen Stapel aus einer ersten metallischen Schicht 121, einer dielektrischen Schicht 11 und einer zweiten metallischen Schicht 12. Dabei ist die erste metallische Schicht 121 auf der Oberfläche 20 der Substratschicht des medizinischen Instruments 2 angeordnet. Die dielektrische Schicht 11 ist auf der ersten metallischen Schicht 121 angeordnet. Die zweite metallische Schicht 12 ist auf der dielektrischen Schicht 11 angeordnet und schließt den Stapel 1 nach oben ab.

Die erste metallische Schicht 121 kann aus Tantal bestehen. Beispielsweise kann die erste metallische Schicht 121 in vertikaler Richtung, d.h. in einer Richtung senkrecht zu der Oberfläche 20 der Substratschicht 2, eine Dicke im Bereich von 10 nm bis 90 nm, wie z.B. im Bereich von 30 nm bis 70 nm aufweisen.

Die dielektrische Schicht 11 besteht in einer Ausführungsform aus Titandioxid (TiO₂). Beispielsweise kann eine vertikale Dicke der dielektrischen Schicht 11 im Bereich von 55 nm bis 65 nm, wie z.B. im Bereich von 57 nm bis 63 nm liegen.

Die zweite metallische Schicht 12 besteht in einer Ausführungsform aus Titan. Beispielsweise kann eine vertikale Dicke der zweiten metallischen Schicht 12 im Bereich von 7 nm bis 13 nm, wie z.B. im Bereich von 8 nm bis 12 nm liegen.

In einer Ausführungsform besteht die erste metallische Schicht 121 aus Tantal und ist 50 nm stark, die dielektrische Schicht 11 besteht aus Titandioxid und ist 60 nm stark, und die zweite metallische Schicht 12 besteht aus Titan und ist 10 nm stark.

In dem in Figur 1A gezeigten ersten Zustand hat die Oberflächenbeschichtung 1 ein erstes spektrales Reflexionsvermögen, d.h. eine charakteristische Wellenlängenabhängigkeit der Reflexion von Licht im optischen Bereich. So ergibt sich in dem ersten Zustand bei Beleuchtung der Oberflächenbeschichtung 1 mit weißem Licht für das menschliche Auge ein erster Farbeindruck. Wenn die zweite metallische Schicht 12 beispielsweise eine 10 nm starke Titanschicht, die dielektrische Schicht 11 eine 60 nm starke Titandioxidschicht und die erste metallische Schicht eine 50 nm starke Tantalschicht ist, ergibt sich in Folge des eingestellten Interferenzverhaltens und einer starken Reflexion an der ersten metallischen Schicht 121 ein blauer Farbeindruck. Dabei kann die zweite metallische Schicht mit der genannten Stärke in dem ersten Zustand bereits weitgehend transparent sein, wobei der Farbeindruck im Wesentlichen durch die Stärke der dielektrischen Schicht bestimmt wird.

Wenn nun das medizinische Instrument 2 - entgegen seiner Bestimmung - zum erneuten Gebrauch durch eine Sterilisationsbehandlung klinisch aufbereitet wird, wird dadurch die Oberflächenbeschichtung 1 irreversibel in einen in Figur 1B dargestellten zweiten Zustand überführt. Beispielsweise kann die Oberflächenbeschichtung 1 im Rahmen der Sterilisationsbehandlung einer Base ausgesetzt werden. In der klinischen Praxis sind Reprozessierungen mit pH-Werten von 10 oder mehr üblich. Wenn die Oberflächenbeschichtung 1 einer Base mit pH > 10 ausgesetzt wird, wird die abdeckende zweite Metallschicht 12 durch Oxidation irreversibel strukturell verändert. Wenn die zweite Metallschicht 12 aus Titan besteht, entsteht auf diese Weise eine abdeckende Schicht 12' aus Titandioxid, wodurch die zweite metallische Schicht 12 in eine transparente dielektrische Schicht 12' überführt wird. In Kombination mit der darunter angeordneten dielektrischen Schicht 11, welche in dem Ausführungsbeispiel ebenfalls aus Titandioxid besteht, wird so in dem zweiten Zustand eine beispielsweise ca. 70 nm starke Titandioxidschicht als oberste Schicht der Oberflächenbeschichtung 1 ausgebildet. Entsprechend dieser neuen Schichtdicke ergibt sich in dem zweiten Zustand ein anderer Farbeindruck als zuvor. Dabei entspricht die neue Schichtdicke z. B. - unter Berücksichtigung des Brechungsindex von Titandioxid, welcher in etwa 2,4 beträgt und den optischen Weg um einen entsprechenden Faktor verlängert - einer Viertel-Wellenlänge roten Lichts. Rote Lichtanteile, die an der Grenzfläche zur abdeckenden Titandioxidschicht 12' reflektiert werden, interferieren mit roten Lichtanteilen, die in die Titandioxidschichten 12', 11 eindringen und an der inneren Grenzfläche zu der ersten metallischen (Tantal-)Schicht 121 reflektiert werden, konstruktiv, denn sie erfahren beim Eintreten in die oberste Titandioxidschicht 12' einen Phasensprung von 180° und durchlaufen danach bis zum Wiederaustreten aus der Titandioxidschicht 12'-unter Berücksichtigung des Brechungsindexes von Titandioxid - insgesamt ca. eine halbe (rote) Wellenlänge. Neben dieser konstruktiven Interferenz können dabei für den sich insgesamt ergebenden Farbeindruck roter Lichtanteile auch destruktive Interferenzen anderer Spektralanteile eine Rolle spielen.

Im Ergebnis ist das spektrale Reflexionsvermögen in dem zweiten Zustand gegenüber dem ersten Zustand dahingehend irreversibel verändert, dass rotes Licht verstärkt reflektiert wird. Die Oberflächenbeschichtung 1 hinterlässt somit in dem zweiten Zustand bei Beleuchtung mit weißem Licht einen roten Farbeindruck. Beispielsweise erscheint somit auf dem medizinischen Instrument 2 in roten Buchstaben der Schriftzug "DO NOT USE".

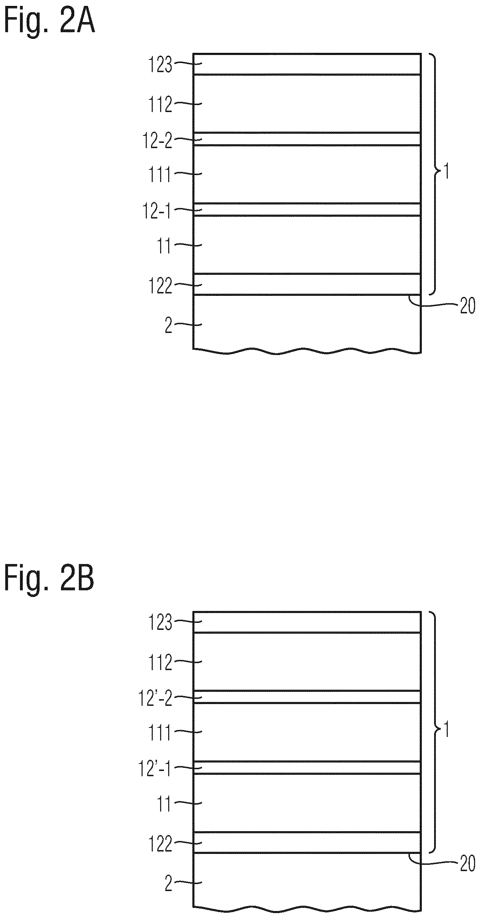

In den Figuren 2A und 2B ist ein zweites Ausführungsbeispiel einer Oberflächenbeschichtung 1 eines medizinischen Instruments 2 dargestellt. Dabei zeigt Figur 2A einen Ausschnitt einer vertikalen Querschnittsansicht der Oberflächenbeschichtung 1 in einem ersten Zustand.

Das medizinische Instrument 2 kann in dieser Ausführungsform beispielsweise ein chirurgisches Handinstrument oder ein Teil davon, wie z.B. ein "single-use"-Bohrer sein. Die Oberflächenbeschichtung 1 kann beispielsweise durch maskiertes reaktives Magnetronsputtern auf einer Oberfläche 20 des medizinischen Instruments 2 ausgebildet sein. Beispielsweise wird die Oberfläche 20 des medizinischen Instruments 2 zunächst (d.h. ohne die Oberflächenbeschichtung 1) aus einer Substratschicht aus Edelstahl, wie dem gebräuchlichen Stahl 316L, gebildet.

Die Oberflächenbeschichtung 1 umfasst in dem in Figur 2A gezeigten ersten Zustand einen Stapel aus den folgenden Schichten: Eine auf der Oberfläche 20 des medizinischen Instruments 2 angeordnete erste metallische Schicht 122; eine erste dielektrische Schicht 11; eine zweite metallische Schicht 12-1, eine zweite dielektrische Schicht 111; eine dritte metallische Schicht 12-2; eine dritte dielektrische Schicht 112; und eine vierte metallische Schicht 123. Dabei sind diese Schichten in der Reihenfolge ihrer Nennung, von der Oberfläche 20 des medizinischen Instruments 2 ausgehend, aufeinander angeordnet, so dass die vierte metallische Schicht 123 den Stapel 1 nach oben abschließt.

Die erste metallische Schicht 122 und die vierte metallische Schicht 123 können aus Tantal bestehen. Beispielsweise können die erste metallische Schicht 122 und die vierte metallische Schicht 123 jeweils eine vertikale Dicke im Bereich von 25 nm bis 35 nm, wie z.B. im Bereich von 28 nm bis 32 nm haben.

Die erste dielektrische Schicht 11, die zweite dielektrische Schicht 111 sowie die dritte dielektrische Schicht 112 können beispielsweise aus Titandioxid bestehen. Eine vertikale Schichtdicke der ersten dielektrischen Schicht 11, der zweiten dielektrischen Schicht 111 und der dritten dielektrischen Schicht 112 liegt beispielsweise jeweils im Bereich von 60 nm bis 70 nm, wie z.B. im Bereich von 63 nm bis 67 nm.

Die zweite metallische Schicht 12-1 und die dritte metallische Schicht 12-2 bestehen in einer Ausführungsform jeweils aus Titan. Beispielsweise kann eine vertikale Dicke der zweiten metallischen Schicht 12-1 und der dritten metallischen Schicht 12-2 jeweils im Bereich von 10 nm bis 20 nm, wie z.B. im Bereich von 13 nm bis 17 nm, liegen.

In einer Ausführungsform betragen die vertikalen Dicken der ersten metallischen Schicht 122 und der vierten metallischen Schicht 123 jeweils 30 nm, die der ersten dielektrische Schicht 11, der zweiten dielektrischen Schicht 111 sowie der dritten dielektrischen Schicht 112 jeweils 65 nm und die der zweiten metallischen Schicht 12-1 und der dritten metallischen Schicht 12-2 jeweils 15 nm. Dabei bestehen die erste metallische Schicht 122 und die vierte metallische Schicht 123 aus Tantal, die erste dielektrische Schicht 11, die zweite dielektrische Schicht 111 und die dritte dielektrische Schicht 112 aus Titandioxid, und die zweite metallische Schicht 12-1 und die dritte metallische Schicht 12-2 bestehen jeweils aus Titan.

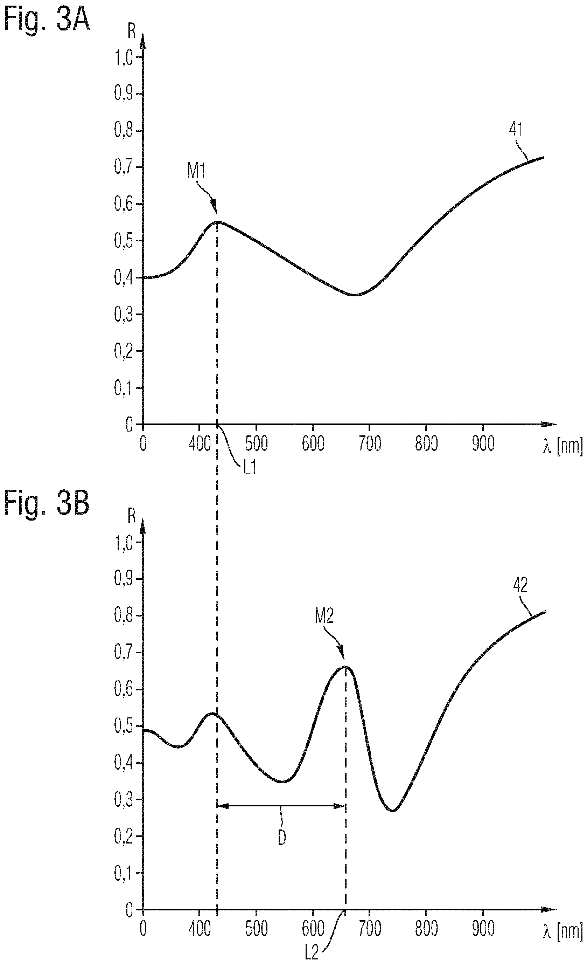

Die Figur 3A zeigt eine grafische Darstellung des ersten spektralen Reflexionsvermögens 41 der Oberflächenbeschichtung 1 aus Figur 2A für das vorstehend beschriebene Ausführungsbeispiel, in welchem: die erste metallische Schicht 122 und die vierte metallische Schicht 123 aus Tantal bestehen und jeweils eine Stärke von 30 nm haben; die erste dielektrische Schicht 11, die zweite dielektrische Schicht 111 sowie die dritte dielektrische Schicht 112 aus Titandioxid bestehen und jeweils eine Stärke von 65 nm haben; und die zweite metallische Schicht 12-1 und die dritte metallische Schicht 12-2 aus Titan bestehen und jeweils 15 nm stark sind. Dabei ist als spektrales Reflexionsvermögen 41 das Verhältnis R von der reflektierten Leistung zu der eingestrahlten Leistung über der Wellenlänge λ in nm aufgetragen. Die Kurve 41 ist somit für das Interferenzfilter der Oberflächenbeschichtung 1 in dem ersten Zustand charakteristisch und stellt das erste spektrale Reflexionsvermögen dar. Das erste spektrale Reflexionsvermögen 41 weist bei einer ersten Wellenlänge L1, die ein wenig oberhalb von 400 nm liegt, ein erstes Reflexionsmaximum M1 auf. Das erste Reflexionsmaximum M1 ist dabei das Maximum des Reflexionsvermögens 41 im sichtbaren Spektrum. Dies entspricht also einer bevorzugten Reflexion für Licht im grün-blauen Spektralbereich. Insgesamt ergibt sich aufgrund des ersten spektralen Reflexionsvermögens 41 in dem ersten Zustand ein blauer Farbeindruck. Dieser ist vom gewählten Betrachtungswinkel (zur Oberflächenbeschichtung 1) weitgehend unabhängig. Damit ist gemeint, dass der Farbeindruck stabil gegenüber einem Wechsel des Betrachtungswinkels wenigstens in einem Bereich zwischen 0° und 60° ist, wobei 0° einer senkrechten Blickrichtung auf die Oberflächenbeschichtung 1 entspricht.

Wenn die Oberflächenbeschichtung 1 aus Figur 2A bei einer klinischen Aufbereitung einem Autoklavierprozess und/oder einer Behandlung mit einer Base, z.B. mit einer Base mit pH > 10, ausgesetzt wird, so werden die zweite metallische Schicht 12-1 und die dritte metallische Schicht 12-2 oxidiert. Dabei kann die Base durch Pinholes, die beim Erzeugen der darüber liegenden Schichten automatisch entstehen, zu der zweiten metallischen Schicht 12-1 und zu der dritten metallischen Schicht 12-2 gelangen. In einer Variante können zu diesem Zweck aber auch definierte Zugänge in den darüber liegenden Schichten strukturiert sein. In einer Ausführungsform werden somit durch Oxidation aus den metallischen Titanschichten 12-1, 12-2 dielektrische und transparente Titandioxidschichten 12'-1, 12'-2. Dies ist in der Figur 2B gezeigt, die den zweiten Zustand der Oberflächenbeschichtung 1 nach der Sterilisationsbehandlung illustriert.

Durch das Interferenzverhalten der Titandioxidschichten 12'-1, 12'-2 hat das Interferenzfilter der Oberflächenbeschichtung 1 in dem zweiten Zustand ein geändertes, zweites spektrales Reflexionsvermögen 42, welches in der Figur 3B grafisch aufgetragen ist. Das zweite spektrale Reflexionsvermögen 42 weist deutlich erkennbar ein zweites Reflexionsmaximum M2 auf. Das zweite Reflexionsmaximum M2 liegt bei einer zweiten Wellenlänge L2 im roten Bereich, in etwa bei 650 nm. Die Differenz D zwischen der ersten Wellenlänge L2 und der zweiten Wellenlänge L1 beträgt somit mehr als 200 nm. So ergibt sich (wiederum weitgehend unabhängig vom Betrachtungswinkel) in dem zweiten Zustand insgesamt ein orange-roter Farbeindruck. Die orange-rote Farbe des medizinischen Instruments 2 zeigt demnach an, dass es einer klinischen Aufbereitung unterzogen wurde.

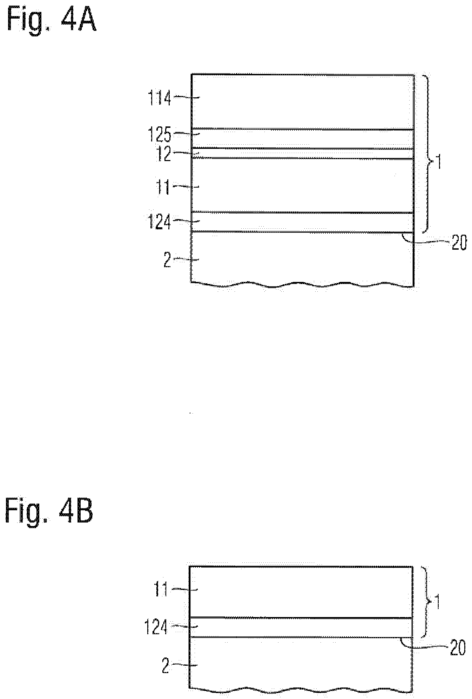

In den Figuren 4A und 4B ist eine weitere Ausführungsform dargestellt, wobei das medizinische Instrument 2, wie bereits mit Bezug auf die Figuren 2A und 2B beschrieben, ein chirurgisches Handinstrument oder ein Teil davon, wie z.B. ein "single-use"-Bohrer, sein kann. Die Oberfläche 20 des medizinischen Instruments 2 kann dabei z.B. aus einer Substratschicht aus Edelstahl, wie dem gebräuchlichen Stahl 316L, gebildet sein. Alternativ kann die Substratschicht 2 z.B. aus der Cobalt-Chrom-Legierung CoCr28Mo6 bestehen.

Die Oberflächenbeschichtung 1 umfasst in dem ersten Zustand gemäß Figur 4A einen Stapel aus den folgenden Schichten: Eine auf der Oberfläche 20 angeordnete erste metallische Schicht 124; eine erste dielektrische Schicht 11; eine zweite metallische Schicht 12, eine dritte metallische Schicht 125; und eine zweite dielektrische Schicht 114. Diese Schichten sind in der Reihenfolge ihrer Nennung, von der Oberfläche 20 des medizinischen Instruments 2 ausgehend, aufeinander angeordnet, so dass die zweite dielektrische Schicht 114 den Stapel 1 nach oben abschließt.

Die erste metallische Schicht 124 und die dritte metallische Schicht 125 aus können aus Tantal bestehen. Beispielsweise können die erste metallische Schicht 124 und die dritte metallische Schicht 125 jeweils eine Dicke im Bereich von 10 nm bis 90 nm, wie z.B. im Bereich von 30 nm bis 70 nm, haben.

Die erste dielektrische Schicht 11 sowie die zweite dielektrische Schicht 114 können aus Titandioxid bestehen. Eine vertikale Schichtdicke der ersten dielektrischen Schicht 11 liegt beispielsweise im Bereich von 270 nm bis 310 nm, wie z.B. im Bereich von 280 nm bis 300 nm. Eine vertikale Schichtdicke der zweiten dielektrischen Schicht 114 kann im Bereich von 140 nm bis 150 nm, z.B. im Bereich von 143 nm bis 147 nm liegen.

Die zweite metallische Schicht 12 besteht in einer Ausführungsform aus Magnesium. Beispielsweise kann eine vertikale Dicke der zweiten metallischen Schicht 12 im Bereich von 5 nm bis 50 nm, wie z.B. im Bereich von 10 nm bis 30 nm, liegen.

Bei einem Ausführungsbeispiel bestehen die erste metallische Schicht 124 und die dritte metallische Schicht 125 jeweils aus Tantal mit einer Stärke von 50 nm, und die erste dielektrische Schicht 11 und die zweite dielektrische Schicht 114 bestehen jeweils aus Titandioxid, wobei die erste dielektrische Schicht 11 290 nm stark ist und die zweite dielektrische Schicht 114 145 nm stark ist. Dabei besteht die zweite metallische Schicht 12 aus Magnesium und ist 10 nm stark.

Die Figur 4B zeigt einen zweiten Zustand, in den die Oberflächenbeschichtung 1 bei einer Sterilisationsbehandlung des medizinischen Instruments 2 irreversibel überführt wird. Beispielsweise kann die Oberflächenbeschichtung 1 im Rahmen der Sterilisationsbehandlung einer Behandlung mit einer Base, z.B. mit einer Base mit pH>10, und/oder einem Autoklavierprozess unterzogen werden. In der klinischen Praxis sind beispielsweise Autoklavierprozesse, d.h. Sterilisationsbehandlungen mit feuchter Hitze, bei Temperaturen im Bereich von 121 °C bis 135 °C für eine Dauer von 1 bis 30 Minuten und Absolutdruck von 1 bis 5 bar üblich. Wenn die Oberflächenbeschichtung 1 solchen Bedingungen ausgesetzt ist, oxidiert die zweite metallische Schicht 12 und delaminiert in der Folge von der ersten dielektrischen Schicht 11. In einem Spülvorgang im Rahmen der Sterilisationsbehandlung kann sodann der obere Teilstapel, bestehend aus der (oxidierten) zweiten metallischen Schicht 12, der dritten metallischen Schicht 125 und der zweiten dielektrischen Schicht 114 irreversibel von der ersten dielektrischen Schicht 11 abgelöst und entfernt werden.

Durch das Ablösen des oberen Teilstapels ändert sich das spektrale Reflexionsvermögen der Oberflächenbeschichtung 1, so dass ähnlich wie bei dem vorstehend mit Bezug auf die Figuren 2A-3B erläuterten Ausführungsbeispiel in Folge der Sterilisationsbehandlung eine für das menschliche Auge deutlich wahrnehmbare Farbänderung vom grünen in den roten Bereich erfolgen kann.

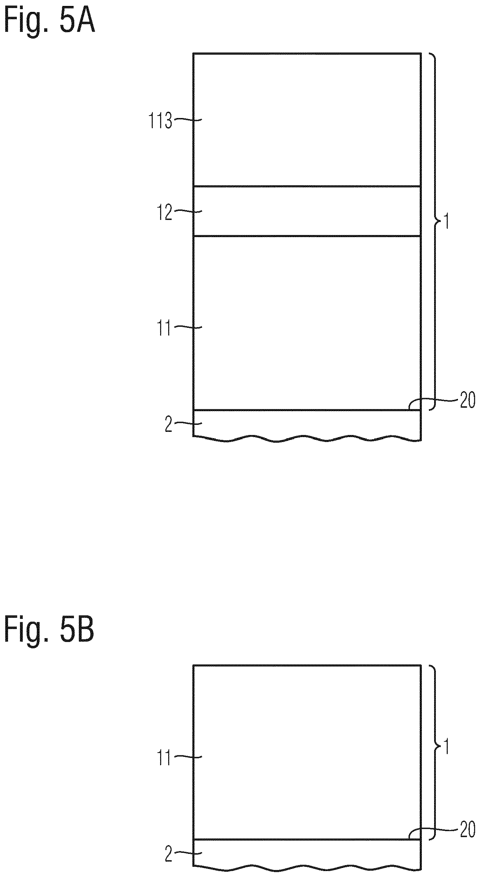

Die Figur 5A zeigt einen Ausschnitt einer vertikalen Querschnittsansicht einer Oberflächenbeschichtung 1 für ein medizinisches Instrument 2 gemäß einem vierten Ausführungsbeispiel. Das medizinische Instrument 2 kann dabei ein chirurgisches Handinstrument wie z.B. ein "single-use"-Skalpell sein. Die Oberflächenbeschichtung 1 ist auf einer Oberfläche 20 des medizinischen Instruments 1 angeordnet. Die Oberfläche 20 kann beispielsweise von einer Substratschicht aus einem üblichen Edelstahl, wie z.B. Stahl 316L, ausgebildet sein.

Die Oberflächenbeschichtung 1 kann beispielsweise durch reaktives Lichtbogenverdampfen auf der Oberfläche 20 erzeugt worden sein. Dies kann in einem maskierten Prozess erfolgen, so dass die Oberflächenbeschichtung 1 z.B. ringförmig umlaufend um den Griff des Skalpells 2 aufgebracht werden kann.

In dem in der Figur 5A dargestellten ersten Zustand umfasst die Oberflächenbeschichtung 1 einen Stapel aus einer ersten dielektrischen Schicht 11, einer metallischen Schicht 12 und einer zweiten dielektrischen Schicht 113. Dabei ist die erste dielektrische Schicht 11 auf der Oberfläche 20 der Substratschicht des medizinischen Instruments 2 angeordnet. Die metallische Schicht 12 ist auf der ersten dielektrischen Schicht 11 angeordnet. Die zweite dielektrische Schicht 113 ist auf der metallischen Schicht 12 angeordnet und schließt den Stapel 1 nach oben hin ab.

In einer Ausführungsform besteht die erste dielektrische Schicht 11 aus Titandioxid. Beispielsweise kann die erste dielektrische Schicht 11 in vertikaler Richtung eine Dicke im Bereich von 105 nm bis 125 nm, wie z.B. im Bereich von 110 nm bis 120 nm aufweisen.

Die metallische Schicht 12 besteht in einer Ausführungsform aus Magnesium. Beispielsweise kann eine vertikale Schichtdicke der metallischen Schicht 12 im Bereich von 5 nm bis 50 nm, wie z.B. im Bereich von 10 nm bis 30 nm liegen.

Die zweite dielektrische Schicht 113 besteht in einer Ausführungsform ebenfalls aus Titandioxid. Beispielsweise kann eine vertikale Dicke der zweiten dielektrischen Schicht 113 im Bereich von 135 nm bis 155 nm, wie z.B. im Bereich von 140 nm bis 150 nm liegen.

In einer Ausführungsform besteht die erste dielektrische Schicht 11 aus Titandioxid mit einer Stärke von 115 nm, die metallische Schicht 12 besteht aus Magnesium mit einer Stärke von 20 nm, und die zweite dielektrisch Schicht 113 besteht aus Titandioxid mit einer Stärke von 145 nm.

In dem in Figur 5A gezeigten ersten Zustand weist die Oberflächenbeschichtung 1 ein erstes spektrales Reflexionsvermögen auf. Wenn z.B. die erste dielektrische Schicht 11 eine 115 nm starke Titandioxidschicht, die metallische Schicht 12 eine 20 nm starke Magnesiumschicht und die zweite dielektrische Schicht 113 eine 145 nm starke Titandioxidschicht ist, ergibt sich bei Betrachtung der Oberflächenbeschichtung 1 unter weißem Licht ein grüner Farbeindruck. Dies beruht auf der konstruktiven und destruktiven Interferenz der Lichtanteile, die an der äußeren Grenzfläche der zweiten dielektrischen Schicht 113 reflektiert werden mit grünen Lichtanteilen, die (unter einem Phasensprung von 180°) in die zweite dielektrische Schicht 113 eindringen und an der inneren Grenzfläche zu der metallischen Schicht 12 reflektiert werden.

Wird das medizinische Instrument 2 durch eine Sterilisationsbehandlung klinisch aufbereitet, so wird dadurch die Oberflächenbeschichtung 1 irreversibel in den in Figur 5B dargestellten zweiten Zustand überführt. Beispielsweise kann die Oberflächenbeschichtung 1 im Rahmen der Sterilisationsbehandlung einem Autoklavierprozess unterzogen werden. In der klinischen Praxis sind beispielsweise Autoklavierprozesse, d.h. Sterilisationsbehandlungen mit feuchter Hitze, bei Temperaturen im Bereich von 121 °C bis 135 °C für eine Dauer von 1 bis 30 Minuten bei einem Absolutdruck von 1 bis 5 bar üblich. Wenn die Oberflächenbeschichtung 1 solchen Bedingungen ausgesetzt ist, oxidiert die metallische Schicht 12 und delaminiert in der Folge von der ersten dielektrischen Schicht 11. In einem Spülvorgang im Rahmen der Sterilisationsbehandlung kann sodann der obere Teilstapel aus der (oxidierten) metallischen Schicht 12 und der zweiten dielektrischen Schicht 113 irreversibel von der ersten dielektrischen Schicht 11 abgelöst und entfernt werden. Im Ergebnis hat die Oberflächenbeschichtung 1 gemäß der Figur 5B in dem zweiten Zustand ein zweites spektrales Reflexionsvermögen, welches bei Beleuchtung mit weißem Licht in einem roten Farbeindruck resultiert.

Es sollte beachtet werden, dass die verschiedenen in den Figuren 1A bis 2B und 3A bis 5B gezeigten metall-dielektrischen Stapel 1 hinsichtlich der einzelnen Schichtdicken nicht notwendigerweise maßstabsgetreu dargestellt sind. Über die vorstehenden beispielhaften Angaben zu den Schichtdicken hinaus sind auch abweichende Schichtdicken denkbar. Die Interferenzfarben einzelner transparenter Schichten, welche sich jeweils durch konstruktive und destruktive Interferenz an dem Interferenzfilter ergeben, können dabei in an sich bekannter Weise durch entsprechende Auslegung der Schichtdicken eingestellt und zu diesem Zweck beispielsweise auch simuliert werden.

Die zuvor beschriebenen Ausführungsbeispiele von Oberflächenbeschichtungen können in dieser oder ähnlicher Weise beispielsweise auch bei einer mikrofluidische Vorrichtung, wie z.B. eine sogenannte "Flow Cell", zur Anwendung kommen. Derartige mikrofluidische Vorrichtungen werden häufig im Rahmen von molekularmedizinischen Verfahren für diagnostische Zwecke, wie z.B. bei der DNA-Sequenzierung, eingesetzt. Beim erstmaligen bestimmungsgemäßen Gebrauch und/oder bei einer Sterilisationsbehandlung einer derartigen mikrofluidischen Vorrichtung kann die Oberflächenbeschichtung einem korrosiven Umgebung ausgesetzt sein und so von einem ersten in einen zweiten Zustand überführt werden.

### Bezugszeichenliste

- 1: Oberflächenbeschichtung
- 11: Dielektrische Schicht
- 111, 112, 113, 114: Weitere dielektrische Schichten
- 12, 12-1, 12-2: Metallische Schicht
- 12', 12'-1, 12'-2: (Vormals) metallische Schicht nach Sterilisationsbehandlung
- 121, 122, 123, 124, 125: Weitere metallische Schichten
- 2: Medizinisches Instrument
- 20: Oberfläche
- 41: Erstes spektrales Reflexionsvermögen
- 42: Zweites spektrales Reflexionsvermögen

- D: Differenz
- L1: Erste Wellenlänge
- L2: Zweite Wellenlänge
- M1: Erstes Reflexionsmaximum
- M2: Zweites Reflexionsmaximum

## Patentansprüche

1. Medizinisches Instrument (2) mit einer Oberflächenbeschichtung (1), umfassend ein Interferenzfilter mit wenigstens einer dielektrischen Schicht (11) und wenigstens einer metallischen Schicht (12, 12-1, 12-2), die übereinander angeordnet sind, wobei:
- die Oberflächenbeschichtung (1) in einem ersten Zustand ein erstes spektrales Reflexionsvermögen (41) aufweist;
- die wenigstens eine metallische Schicht (12, 12-1, 12-2) und/oder die wenigstens eine dielektrische Schicht (11) ausgebildet ist, durch den Einfluss einer korrosiven Umgebung auf die Oberflächenbeschichtung (1) strukturell verändert zu werden, wodurch die Oberflächenbeschichtung (1) in einen zweiten Zustand überführbar ist; und
- die Oberflächenbeschichtung (1) in dem zweiten Zustand ein zweites spektrales Reflexionsvermögen (42), welches von dem ersten spektralen Reflexionsvermögen (41) verschieden ist, aufweist,
- das Interferenzfilter eine Mehrzahl von dielektrischen Schichten (11, 111, 112, 113, 114) und/oder eine Mehrzahl von metallischen Schichten (12, 121, 122, 123, 124, 125) umfasst.

2. Medizinisches Instrument (2) mit einer Oberflächenbeschichtung (1) nach Anspruch 1, wobei die Oberflächenbeschichtung (1) bei Beleuchtung mit weißem Licht in dem ersten Zustand eine erste Farbe und in dem zweiten Zustand eine zweite Farbe hat, wobei die zweite Farbe von der ersten Farbe verschieden ist.

3. Medizinisches Instrument (2) mit einer Oberflächenbeschichtung (1) nach einem der vorangehenden Ansprüche, wobei
- das erste spektrale Reflexionsvermögen (41) ein erstes Reflexionsmaximum (M1) bei einer ersten Wellenlänge (L1) aufweist und
- das zweite spektrale Reflexionsvermögen (42) ein zweites Reflexionsmaximum (M2) bei einer zweiten Wellenlänge (L2) aufweist,
wobei eine Differenz (D) zwischen der zweiten Wellenlänge (L2) und der ersten Wellenlänge (L1) dem Betrag nach wenigstens 100 nm ist.

4. Medizinisches Instrument (2) mit einer Oberflächenbeschichtung (1) nach einem der vorangehenden Ansprüche, wobei die wenigstens eine metallische Schicht (12) ausgebildet ist, durch den Einfluss der korrosiven Umgebung oxidiert und/oder von einer anderen Schicht (11) der Oberflächenbeschichtung (1) abgelöst zu werden, wodurch die Oberflächenbeschichtung (1) in den zweiten Zustand überführbar ist.

5. Medizinisches Instrument (2) mit einer Oberflächenbeschichtung (1) nach einem der vorangehenden Ansprüche, wobei die mindestens eine metallische Schicht (12, 12-1, 12-2) aus Titan und/oder Magnesium besteht.

6. Medizinisches Instrument (2) mit einer Oberflächenbeschichtung (1) nach einem der vorangehenden Ansprüche, wobei die mindestens eine dielektrische Schicht (11) aus Titandioxid besteht.

7. Medizinisches Instrument (2) mit einer Oberflächenbeschichtung (1) nach einem der vorangehenden Ansprüche, wobei die Oberflächenbeschichtung (1) durch eine Sterilisationsbehandlung, bei welcher die Oberflächenbeschichtung (1) der korrosiven Umgebung ausgesetzt ist, in den zweiten Zustand überführbar ist.

8. Medizinisches Instrument (2) mit einer Oberflächenbeschichtung (1) nach Anspruch 7, wobei die Sterilisationsbehandlung ein Autoklavierprozess und/oder eine Behandlung der Oberflächenbeschichtung (1) mit einer Base ist.

9. Medizinisches Instrument (2) mit einer Oberflächenbeschichtung (1) nach einem der vorangehenden Ansprüche, wobei die mindestens eine metallische Schicht (12, 12-1, 12-2) ausgebildet ist, durch den Einfluss der korrosiven Umgebung ihre optische Transparenz zu erhöhen oder von einem optisch intransparenten Zustand in einen optisch transparenten Zustand überführt zu werden.

10. Medizinisches Instrument (2) nach einem der vorhergehenden Ansprüche, wobei das medizinische Instrument (2) wenigstens eines der Folgenden ist:
- ein Handinstrument;
- eine Vorrichtung zur Implantation in den menschlichen oder tierischen Körper;
- eine mikrofluidische Vorrichtung.

## Claims

1. Medical instrument (2) having a surface coating (1), comprising an interference filter having at least one dielectric layer (11) and at least one metallic layer (12, 12-1, 12-2) arranged one above another, wherein:
- in a first state the surface coating (1) has a first spectral reflectivity (41);
- the at least one metallic layer (12, 12-1, 12-2) and/or the at least one dielectric layer (11) are/is configured to be structurally altered by the influence of the corrosive environment on the surface coating (1), as a result of which the surface coating (1) is able to be converted into a second state; and
- in the second state the surface coating (1) has a second spectral reflectivity (42), which is different from the first spectral reflectivity (41),
- the interference filter comprises a plurality of dielectric layers (11, 111, 112, 113, 114) and/or a plurality of metallic layers (12, 121, 122, 123, 124, 125).

2. Medical instrument (2) having a surface coating (1) according to Claim 1, wherein, in the case of illumination with white light, the surface coating (1) has a first colour in the first state and a second colour in the second state, wherein the second colour is different from the first colour.

3. Medical instrument (2) having a surface coating (1) according to either of the preceding claims, wherein
- the first spectral reflectivity (41) has a first reflection maximum (M1) at a first wavelength (L1) and
- the second spectral reflectivity (42) has a second reflection maximum (M2) at a second wavelength (L2),
wherein a difference (D) between the second wavelength (L2) and the first wavelength (L1) in terms of absolute value is at least 100 nm.

4. Medical instrument (2) having a surface coating (1) according to any of the preceding claims, wherein the at least one metallic layer (12) is configured to be oxidized and/or detached from another layer (11) of the surface coating (1) as a result of the influence of the corrosive environment, as a result of which the surface coating (1) is able to be converted to the second state.

5. Medical instrument (2) having a surface coating (1) according to any of the preceding claims, wherein the at least one metallic layer (12, 12-1, 12-2) consists of titanium and/or magnesium.

6. Medical instrument (2) having a surface coating (1) according to any of the preceding claims, wherein the at least one dielectric layer (11) consists of titanium oxide.

7. Medical instrument (2) having a surface coating (1) according to any of the preceding claims, wherein the surface coating (1) is able to be converted to the second state by a sterilization treatment in which the surface coating (1) is exposed to the corrosive environment.

8. Medical instrument (2) having a surface coating (1) according to Claim 7, wherein the sterilization treatment is an autoclaving process and/or a treatment of the surface coating (1) with a base.

9. Medical instrument (2) having a surface coating (1) according to any of the preceding claims, wherein the at least one metallic layer (12, 12-1, 12-2) is configured to increase its optical transparency or to be converted from an optically non-transparent state to an optically transparent state as a result of the influence of the corrosive environment.

10. Medical instrument (2) according to any of the preceding claims, wherein the medical instrument (2) is at least one of the following:
- a hand-held instrument;
- a device for implantation into a human or animal body;
- a microfluidic device.

## Revendications

1. Instrument médical (2) pourvu d'un revêtement de surface (1), comprenant un filtre antiparasite ayant au moins une couche diélectrique (11) et au moins une couche métallique (12, 12-1, 12-2) qui sont disposées l'une au-dessus de l'autre,
- le revêtement de surface (1) présentant, dans un premier état, une première réflectivité spectrale (41) ;
- l'au moins une couche métallique (12, 12-1, 12-2) et/ou l'au moins une couche diélectrique (11) étant configurées pour être modifiées structurellement par l'influence d'un environnement corrosif sur le revêtement de surface (1), moyennant quoi le revêtement de surface (1) peut être converti dans un deuxième état, et
- le revêtement de surface (1) présentant, dans un deuxième état, une deuxième réflectivité spectrale (42) qui est différente de la première réflectivité spectrale (41),
- le filtre antiparasite comportant une pluralité de couches diélectriques (11, 111, 112, 113, 114) et/ou une pluralité de couches métalliques (12, 121, 122, 123, 124, 125) .

2. Instrument médical (2) pourvu d'un revêtement de surface (1) selon la revendication 1, le revêtement de surface (1), lors d'un éclairage avec de la lumière blanche, ayant une première couleur dans le premier état et une deuxième couleur dans le deuxième état, la première couleur étant différente de la deuxième couleur.

3. Instrument médical (2) pourvu d'un revêtement de surface (1) selon l'une des revendications précédentes,
- la première réflectivité spectrale (41) possédant un premier maximum de réflexion (M1) à une première longueur d'onde (L1) et
- la deuxième réflectivité spectrale (42) possédant un deuxième maximum de réflexion (M2) à une deuxième longueur d'onde (L2),
une différence (D) entre la deuxième longueur d'onde (L2) et la première longueur d'onde (L1) ayant une valeur absolue d'au moins 100 nm.

4. Instrument médical (2) pourvu d'un revêtement de surface (1) selon l'une des revendications précédentes, l'au moins une couche métallique (12) étant configurée pour, sous l'influence de l'environnement corrosif, être oxydée et/ou détachée d'une autre couche (11) du revêtement de surface (1), moyennant quoi le revêtement de surface (1) peut être converti dans le deuxième état.

5. Instrument médical (2) pourvu d'un revêtement de surface (1) selon l'une des revendications précédentes, l'au moins une couche métallique (12, 12-1, 12-2) se composant de titane et/ou de magnésium.

6. Instrument médical (2) pourvu d'un revêtement de surface (1) selon l'une des revendications précédentes, l'au moins une couche diélectrique (11) se composant de dioxyde de titane.

7. Instrument médical (2) pourvu d'un revêtement de surface (1) selon l'une des revendications précédentes, le revêtement de surface (1) pouvant être converti dans le deuxième état par un traitement de stérilisation lors duquel le revêtement de surface (1) est exposé à l'environnement corrosif.

8. Instrument médical (2) pourvu d'un revêtement de surface (1) selon la revendication 7, le traitement de stérilisation étant un processus d'autoclavage et/ou un traitement du revêtement de surface (1) avec une base.

9. Instrument médical (2) pourvu d'un revêtement de surface (1) selon l'une des revendications précédentes, l'au moins une couche métallique (12, 12-1, 12-2) étant configurée pour, sous l'influence de l'environnement corrosif, augmenter sa transparence optique ou être convertie d'un état optiquement intransparent dans un état optiquement transparent.

10. Instrument médical (2) selon l'une des revendications précédentes, l'instrument médical (2) étant au moins l'un des suivants :
- un instrument manuel ;
- un dispositif destiné à être implanté dans les corps humains ou animaux ;
- un dispositif microfluidique.
